Europäisches Patentamt

European Patent Office

Office européen des brevets

(11) Publication number: **0 546 533 A2**

# EUROPEAN PATENT APPLICATION

(21) Application number: **92121019.1**

(22) Date of filing: **09.12.92**

(51) Int. Cl.⁵: **A61K 37/553**, //C12N9/64

(30) Priority: **10.12.91 JP 349905/91**
**13.12.91 JP 351325/91**

(43) Date of publication of application:
**16.06.93 Bulletin 93/24**

(84) Designated Contracting States:
**AT BE CH DE DK ES FR GB GR IE IT LI LU MC NL PT SE**

(71) Applicant: **SANWA KAGAKU KENKYUSHO CO., LTD.**
**No. 35, Higashi-sotobori-cho, Higashi-ku**
**Nagoya, Aichi-ken(JP)**

(72) Inventor: **Sawai, Kiichi**
**35, Higashi-sotobori-cho, Higashi-ku**
**Nagoya, Aichi-ken(JP)**
Inventor: **Kurono, Masayasu**
**35, Higashi-sotobori-cho, Higashi-ku**
**Nagoya, Aichi-ken(JP)**
Inventor: **Hayashi, Motohide**
**35, Higashi-sotobori-cho, Higashi-ku**
**Nagoya, Aichi-ken(JP)**
Inventor: **Suzuki, Tsunemasa**
**35, Higashi-sotobori-cho, Higashi-ku**
**Nagoya, Aichi-ken(JP)**
Inventor: **Suzuki, Tamikazu**
**35, Higashi-sotobori-cho, Higashi-ku**
**Nagoya, Aichi-ken(JP)**
Inventor: **Asano, Osamu**
**35, Higashi-sotobori-cho, Higashi-ku**
**Nagoya, Aichi-ken(JP)**

(74) Representative: **Wächtershäuser, Günter, Dr.**
**Tal 29**
**W-8000 München 2 (DE)**

(54) **Medical use of kininogenase.**

(57) There are disclosed uses of kininogenase as an effective ingredient for improving cerebral functions as well as preventing and curing a skin disease. The kininogenase is extracted from human urine and purified to show a main band at a position in molecular weight of 41,000 and a sub-band in molecular weight of 31,000, when SDS-polyacryloamide gel electrophoresis is applied for, a molecular weight in the range of about 47,000 - about 55,000, when gel-filtration methods are applied for, as well as isoelectric points of pI 3.92, 4.08 and 4.23 in its original solution.

The present invention relates to a use of kininogenase as an effective ingredient for pharmaceutical preparations to improve cerebral functions, and to preventing or cure a skin disease.

In recent years, medical techniques have great advanced to prolong the average span of human life and as a result, a senile dementia and more particularly, that of Alzheimer type and aged peoples who can not leave his bed with own force tend to increase. Further, a rapid change in recent social environment gives an excess stress to persons to cause modern mental diseases such as a retention defect, aphasia, apraxia and the like, and an excessive mental labor and arteriosclerosis induces an allergy to cause skin diseases such as a skin ulcer.

It has been said that the Alzheimer type senile dementia is caused by an atrophy of cerebral nerve cells, but there is no therapeutic method for preventing or curing the same, since a cause of the atrophy has not been elucidated. There is also no drug for curing the modern mental diseases and cerebral dysfunctions due to cerebral infarction, hemorrhage and others, so that, at the present time, an administration of agent for expanding cerebral blood vessels or avan has been inevitably done, by taking symptoms into consideration. In view of such a situation, the present inventors have energetically studied and investigated for finding out a substance which shows an action for improving cerebral functions. As a result, they have found that one of myoinositol compounds, namely phytic acid and a salt thereof is effective for preventing and curing hypofunctional waste-diseases and more particularly, so-called "senile diseases" [Jap. Pat. No. Hei 1 (A.D. 1989) - 305032(A)]. It has also been reported that pyrazolopyridine compounds have an action for improving a retention defect.

While, on the skin diseases inclusive of decubitus (bedsore), it has been considered that those do not give a danger of the patient's life, excepting that an affected part occupies a large area in total surface of his body. For curing the skin diseases, in general, a skin protecting agent, anti-agent to infectional diseases, a steroid hormone such as an aderenocortical hormone or the like has been locally applied. However, each of those belongs to an allopathy and it can be said as that a radical or drastic curing method has not been established. In view of the circumstance, the present inventors have also been studied for finding out a substance which develops its pharmacological action through an oral or injectional administration to cure the skin diseases such as skin ulcer. As a results, they have found that a hydantoin compound as one of inhibitors to aldose reductases is effective for preventing and curing the skin ulcer due to a complication of diabetes [Jap. Pat. No. Hei 3 (A.D. 1991) - 215435(A)]. It has also been reported that a protein obtained from cultured substance of bacterial strain from Clostridium or the like genus shows a cell proliferation accelerating action [Jap. Pat. No. Sho 60 (A.D. 1985) - 28999(A)].

In turning now to the substance of kininogenase to be employed for the pharmaceutical preparations according to the invention as an effective ingredient, it is one of trypsine-like enzyme which has been named as "kininogenin" by the International Committee for Naming Enzymes and also "kaliginogenase" and "kallikrein" and presents in blood plasma and various tissues. It has been said that there are two types of the kininogenase, namely former one in plasma has a molecular weight of about 80,000 to make free bradykinin (Arg-Pro-Pro-Gly-Phe-Ser-Pro-Phe-Arg), and the other one in issue has a molecular weight of about 30,000 to make free kallidin (Lys-Arg-Pro-Pro-Gly-Phe-Ser-Pro-Phe-Arg). The kininogenase biosynthesized mainly in liver is different in its characteristics from that derived in tissues and presenting with relatively high concentration in pancreas, submandibular gland and kidney, and it has been said that among those derived from the tissues, the kininogenases found in the pancreas and submandibular gland are immuno-chemically same with that found in urine, but whether those are entirely same or not has not yet been made apparent.

The kininogenase derived from the tissue decompose kininogen to form kinin and through this, it expands arterioles to increase a blood flow and develops a blood pressure descending action. Therefore, a preparation comprising as an effective ingredient the kininogenase derived from porcine pansreas in view of its availability thereof in markets has been crinically employed for improving dysfunctions in peripheral circulation due to hypertension, Meniere's symptom complex, and thromboangilis obliterans (Burger disease), and for improving accompanying symptom due to dysfunction of cerebral circulation, crimacteric dysfunctions and circulatory dysfunction in choroid. The preparations have generally been administered through oral route, since the enzyme in question is one of heterogeneous proteins to prevent or suppress its behavior as an antigen in a living body, but there are problems in absorption of the drug in living bodies and effectiveness thereof.

Kininogenase derived from human urine has been watched with great interests, since its antigenity shall be considered lower than that in the kininogenase derived from the porcine pancreas, and therefore, various processes for extracting and purifying the same have been proposed [Jap. Pat. Nos. Sho 46 (A.D. 1971) - 19067(B), Hei 1 (A.D. 1989) - 41310(A) and Hei 1 (A.D. 1989) - 47997(A)]. It has not been confirmed whether the kininogenase derived from human urine is a single substance or not, there is no standard thereon, and thus it has not yet been utilized as an effective ingredient for various medicines.

However, there is no report of that any of kininogenase is effective for improving cerebral functions, or for preventing and curing skin diseases, and more particularly a skin ulcer.

One of the objects of the invention is to provide a pharmaceutical preparation for improving cerebral functions to make possible a prevention and curing of various cerebral dysfunctions.

Another object of the invention is to provide a pharmaceutical preparation for preventing and curing a skin diseases such as skin ulcer.

According to the invention, the objects can be attained by a pharmaceutical preparation which comprises as an effective ingredient a kininogenase and more particularly, of that derived from human urine, since such facts have been found by the present inventors that the enzyme shows powerful actions for improving cerebral dysfunctions, and for preventing and curing the skin diseases and is excellent in safety of its use.

The kininogenase to be employed for the invention is characterized in that it is extracted and purified from human urine, has a main band at a position in molecular weight of 41,000 and a sub-band in molecular weight of 31,000, when SDS-polyacryloamide gel electrophoresis is applied for, shows a molecular weight in a range of about 47,000 - about 55,000, when gel-filtration methods are applied for, as well as isoelectric points of pI 3.92, 4.08 and 4.23 in its original solution.

It is preferable that each of the pharmaceutical preparations comprises the kininogenase of about 0.15 PNA units/ml and 0.1 - 1.0% by weight of a substance selected from the group consisting of citric acid and pharmaceutically acceptable salts thereof.

Please note that an activity of the kininogenase is measured by acting the same to H-D-Val-Leu-Arg-p-nitoroanilide as a substrate to make free p-nitroanilide (PNA), and determining an amount of p-nitroanilide through a measurement of absorbance at 405nm, and assuming as 1 PNA unit, when $1\mu$ mol of PNA is formed by 1 minute at $37 \pm 0.1°C$.

The ground of that citric acid or its salt such as sodium or potassium salt is co-existed for the preparations according to the invention lies in that the kininogenase is not so stable to heat and light in similar to general enzymes, namely a reduction in its activity can be found, when it is reserved for 6 months or more at room temperature, and if lightbeam of 2500 lux or more illumination intensity is radiated thereto, the reduction in activity can be recognized within relatively short period of time. In other words, the coexistence is not form an indispensable matter, if the kininogenase shall be reserved under a condition shielding from lightbeam and at a low temperature, for instance, about -10°C.

The invention will now be explained in more detail with reference to an Example for extracting and purifying kininogenase from human urine, Examples for preparing a pharmaceutical preparation, and Pharmacological Test Examples.

The pharmaceutical preparations according to the invention is mainly intended to administer the same with an intravenous injection to suppress its decomposition and improve an absorption and bioavailability thereof, and thus following explanation shall be given thereon.

Example 1 (Preparation of "original solution")

Human urine (10 litres) was taken from a urine sample collected from healthy male persons and pooled in a vessel and its pH was adjusted to 5.0 by adding therein aqueous hydrochloride solution. To the pH adjusted solution, 100g of chitosan were added to stir the solution for 1 hour for causing an adsorption of kininogenase in the urine to chitosan. The chitosan adsorbed therein the kininogenase was obtained through filtration, and sufficiently washed with water, and then 0.2M Toris chloride buffer (500ml, pH 9.0) was added to cause an elution of the kininogenase, an extract of which was obtained through a filtration.

The extract was dialyzed by using 0.1M sodium hydrogen carbonate buffer (pH 9.0) containing 0.5M sodium chloride therein. The resulting dialyzed solution was treated with aprotinin-sepharose column (trypsin inhibitor) to cause adsorption of the kininogenase to the column, and the latter was eluted with 0.1M phosphate buffer (pH 3.5) containing 0.5M sodium chloride to obtain a solution (45 PNA units) which was called as "original solution".

Followings are physico-chemical properties of the original solution.

(1) General properties

A colorless or pale yellowish clear and transparent solution with no smell, a slight salty taste, and pH of about 7.

3

(2) Molecular weight

(A) SDS-Polyacryloamide gel electrophoresis method

(a) Laemmli method

A main band and sub-band were recognized at positions corresponding to molecular weights of 41,000 and 31,000, respectively and both of the substances showed an activity.

(B) Gel-filtration methods

(a) When Sephacryl S-200 is used :

A single peak was recognized at position corresponding to molecular weight of about 50,000, which coincided with a peak in activity.

(b) When Sephadex G-100 is used :

A single peak was recognized at position corresponding to molecular weight of about 47,000, which coincided with an peak in activity.

(c) High speed liquid chromatography using TSK-GEL (marketed by Toso Co., Ltd. of Japan) :

A single peak was recognized at position corresponding to molecular weight of about 51,000 - 55,000 (mean : 53,000 ± 2,000) which coincided with an peak in activity.

(3) Composition of amino acids

Assuming that molecular weight of the kininogenase is 50,000 by taking the measured values as above into consideration, number of amino acid residues encoding the same becomes 375 and composition thereof shall be estimated as shown in following Table 1. As apparently therefrom, amino acids constituting the kininogenase are rich in acidic one in comparison with basic one. A result of tried Edman decomposition method showed that an amino acid sequence at N-terminal is Ile-Val-Gly which is same with a primary structure at N-terminal known for the kininogenase derived from human urine and for the other kininogenase derived from human kidney tissue and provided through cDNA analysis thereof.

Table 1

| Amino acid | Residue | |
| --- | --- | --- |
| | A | B |
| Asp | 43.5 | 44 |
| Thr | 22.6 | 23 |
| Ser | 27.4 | 27 |
| Glu | 47.8 | 48 |
| Pro | 26.2 | 26 |
| Gly | 32.0 | 32 |
| Ala | 19.6 | 20 |
| Cys | 5.6 | * 6 (12) |
| Val | 32.8 | 33 |
| Met | 6.2 | 6 |
| lle | 12.5 | 13 |
| Leu | 33.8 | 34 |
| Tyr | 7.4 | 7 |
| Phe | 13.0 | 13 |
| Lys | 12.4 | 12 |
| His | 17.1 | 17 |
| Arg | 7.7 | 8 |
| Trp | n.d. | – |
| Total | – | 375 |

In Table 1,

A : Number of moles of amino acid in 1 mole of kininogenase (measured value),

B : Integrated value of A,

* : Half-cystine, and

n.d. : Not detected.

(4) Composition of saccharides

The kininogenase is one of acidic glycoproteins and showed a composition of saccharides, as given in following Table 2. It was confirmed that N-glycoside type saccharic chain is connected with O-glycoside type saccharic chain.

Table 2

| Saccharides | Ratio (W/W %) |
|---|---|
| Man. | 3.0 |
| Fuc. | 0.7 |
| Gal. | 1.8 |
| GlcNAc | 5.0 |
| GalNAc | 3.4 |
| Sialic acid | 0.4 |
| Total | 14.3 |

(5) Isoelectric point

Isoelectric point of the original kininogenase solution was measured by carrier-Ampholite containing sucrose concentration-gradient method to find as pI 3.92, 4.08 and 4.23.

(6) Specific activity

The original kininogenase solution showed 1.5 PNA units/ml or more (measured value : more than 2.4 PNA units/ml), which corresponds to 4.0 PNA units or more per 1mg of protein (measured value : more than 5 PNA units/mg).

(7) Substance inhibiting the activity

The activity of kininogenase was inhibited by more than 95% with aprotinin (measured value : more than 98%).

(8) Immunological properties

Immunological properties of the original kininogenase solution were checked by Ouchterlony method to find that the kininogenase causes clear precipitation line with rabbit anti-human urine kininogenase serum, but causes no precipitation line with rabbit anti-porcine pancreas kininogenase serum.

(9) Enzymatic properties

(A) When natural kininogen is used as substrate

Effects of the kininogenase to an ability making free kinin were checked.

In the first place, effects of pH were checked to find that an activity making free the kinin was recognized in pH range of 5 - 11, when human lower molecule kininogen and canine lower molecule kininogen were used as a substrate, and maximal ability was found at pH of about 8.5.

In the next place, An ability of the kininogenase for making free the kinin was periodically checked by using canine lower molecule kininogen, human lower molecule kininogen and human higher molecule kininogen in various concentration, as substrates to calculate enzymatic dynamic constants of the kininogenase. Results are shown in following Table 3.

Table 3

| Items | Human LMWK | Human HMWK | Canine LMWK |
|---|---|---|---|
| Km (x $10^{-6}$ M) | 1.3 | 0.71 | 1.4 |
| $V_{max}$ (x $10^{-6}$ M/min/mg) | 6.7 | 4.6 | 3.0 |
| Kcat ($S^{-1}$) | 5.6 | 3.8 | 2.5 |
| In Table 3,<br>LMWK : Lower molecule kininogen, and<br>HMWK : Higher molecule kininogen. | | | |

(B) When lower molecule synthetic substrates are used

An activity of the kininogenase to decomposition of lower molecule synthetic substrates was checked as in said Item (A) to calculate enzymatic dynamic constants of the kininogenase. Results are shown in following Table 4.

Table 4

| Item | BAEE | PNA | MCA |
|---|---|---|---|
| Optimum pH | 9.0 - 10.0 | 10.5 | 11.5 |
| Km (M) | 2.52 x 10-4 | 8.95 x 10-6 | 3.4 x 10-5 |
| Vmax (x 10-6/min/mg) | 42.3 | 7.80 | 25.7 |
| In Table 4,<br>BAEE : Benzoyl-L-Arg ethyl ether hydrochloride,<br>PNA : H-D-Val-L-Leu-L-Arg-p-nitroanilide dihydrochloride, and<br>MCA : L-Pro-L-Phe-L-Arg-4-methyl-cumaryl-7-amide. | | | |

Example 2 (Production of stabilized powder)

To the original solution obtained by Example 1, citric acid was added to make a concentration of the latter becomes 0.5%. The resulting solution was heated for 10 hours at 60°C to sterilize the same, and then subjected to lyophilization to obtain a desired stabilized powder.

Example 3 (Ampuled product)

The lyophilized powder obtained by Example 2, saline was adjusted to prepare solutions containing 0.15 PNA units/ml of the kininogenase. Each of the solutions was aseptically charged in ampules by 0.15, 1.0 or 2.0ml, which ampules were then sealed to obtain desired ampuled products.

When it shall be used, the ampule is opened and the solution was diluted with 100ml of a supplemental solution (such as "Fishsartz", "Sorita T3" or "Lactonegel liquid --Fuso--"), to administrate the same by intravenous drip infusion. It is preferable to set the period of time for the infusion to 30 minutes.

Pharmacological Test Example 1

(Action to cerebral circulation metabolism of normal animal)

(1) Blood flow in vertebral artery

An action of the kininogenase to an amount of blood flow in canine vertebral artery was checked by administrating the same into the artery and a vein through a rapid injection (injectional speed in general

7

EP 0 546 533 A2

manual operation) and sustaining injection (drip injection for 30 minutes).

The kininogenase was rapidly administrated at a dose of $1.5 \times 10^{-4}$ - $5.0 \times 10^{-2}$ PNA units/body into the vertebral artery of an anethetized dog to find that a blood flow therein increases in depending to the amount of dose, and its action sustained for 1 - 2 minutes.

In the next place, the kininogenase was rapidly administrated at a dose of $5.0 \times 10^{-5}$ - $1.5 \times 10^{-1}$ PNA units/kg into a vein in upper arm of the animal, or sustainingly administrated at a dose of $5.0 \times 10^{-4}$ - $2.5 \times 10^{-2}$ PNA units/kg.

In case of the rapid administration, an increase of blood flow depending on the dose was recognized in the groups of $1.5 \times 10^{-4}$ - $5.0 \times 10^{-3}$ PNA units/kg administration, but in the groups of $5.0 \times 10^{-2}$ PNA units/kg or more administration, a reduction in blood flow was began after about 1 minute from the administration. While, in case of sustaining administration, an increase of blood flow was not recognized in the groups of $1.5 \times 10^{-3}$ PNA units/kg or less administration, increase of blood flow was sustained during administration period of time on the group of $2.5 \times 10^{-3}$ PNA units/kg administration, reduction tendency in blood flow was recognized by the way of administration on the group of $5.0 \times 10^{-3}$ PNA units/kg administration, and apparent decrease in blood flow was recognized in the group of $2.5 \times 10^{-2}$ PNA units/kg administration.

(2) Blood flow in internal carotid artery

An action of the kininogenase to an amount of blood flow in canine internal carotid artery was checked by administrating the same into the artery through the rapid injection and sustaining injection.

The kininogenase was rapidly administrated at a dose of $1.5 \times 10^{-4}$ - $5.0 \times 10^{-2}$ PNA units/body into the internal carotid artery of an anethetized dog to find that a blood flow therein increase in depending to the amount of dose, and its action sustained for about 1 minute.

In such doses, an increase in blood flow was not recognized, and in cases of rapid administration at dose of $1.5 \times 10^{-2}$ PNA units/kg and sustaining administration of $5.0 \times 10^{-3}$ - $2.5 \times 10^{-2}$ PNA units/kg, a reduction of blood flow was recognized.

(3) Blood flow at local portions in brain

An action of the kininogenase to local portions in canine brain (deep area and cortex portion) was checked.

The kininogenase was rapidly administrated to a vein of an anethetized dog at a dose of $5.0 \times 10^{-5}$ - $2.5 \times 10^{-3}$ PNA units/kg or sustainingly administrated at a dose of $2.5 \times 10^{-3}$ - $2.5 \times 10^{-2}$ PNA units/kg.

In cases of the rapid administrations, an increasing tendency in blood flow was recognized, but in cases of the sustaining administrations, no change in blood flow was recognized in the groups of $2.5 \times 10^{-3}$ - $5.0 \times 10^{-3}$ PNA units/kg administration and a reducing tendency was recognized in the groups of $1.0 \times 10^{-2}$ PNA units/kg or more administration.

(4) Micro-circulation in cerebral pia leptmeninges

The kininogenase was rapidly administrated into a vein of anethetized dog at a dose of $2.5 \times 10^{-3}$ or $5.0 \times 10^{-3}$ PNA units/kg, or sustainingly administrated at a dose of $2.5 \times 10^{-3}$ PNA units/kg, and then a diameter of arterioles in cerebral pia leptmeninges was measured through a photograph method. The diameter was observed by classifying the same into the group of not exceeding $100\mu$ m and another group of $100\mu$ m or more.

An expansion in all of the pia leptmeninges was recognized in the rapid administration groups, but no apparent change in diameter can be confirmed in the sustaining administration group.

(5) Resistance in cerebral blood vessels

The kininogenase was rapidly administrated into a vein of anethetized dog at a dose of $1.5 \times 10^{-4}$ - $2.5 \times 10^{-3}$ PNA units/kg, or sustainingly administrated at a dose of $5.0 \times 10^{-4}$ - $2.5 \times 10^{-2}$ PNA units/kg.

In cases of the rapid administration, resistance in the cerebral blood vessels reduced in depending to an amount of dose, and the action was sustained for about 15 minutes in the group of $2.5 \times 10^{-3}$ PNA units/kg administration. In cases of the sustaining administration, while, a reduction of cerebral blood vessel resistance was recognized in the groups of $2.5 \times 10^{-3}$ PNA units/kg or more administration, and the action sustained also after its administration in the group of $2.5 \times 10^{-2}$ PNA units/kg administration.

8

(6) Difference in an amount of oxygen in cerebral artery and vein

A difference in an amount of oxygen in cerebral artery and vein was checked by rapidly administrating or sustainingly administrating the kininogenase into a vein of anethetized dog, and then taking a blood from vein in upper curare cavity and femoral artery.

There was such a tendency that the difference becomes wide at a dose of $1.5 \times 10^{-4}$ - $2.5 \times 10^{-3}$ PNA units/kg in the rapid administration and $5.0 \times 10^{-4}$ - $5.0 \times 10^{-3}$ PNA units/kg in the sustaining administration.

(7) Concentration of oxygen in cerebral tissue

A concentration of oxygen in deep and cortex portions of the brain was measured by rapidly administrating $1.5 \times 10^{-4}$ - $2.5 \times 10^{-3}$ PNA units/kg) or sustainingly administrating ($5.0 \times 10^{-4}$ - $5.0 \times 10^{-3}$ PNA units/kg the kininogenase into a vein of anethetized dog.

In cases of the rapid administration, there was such a tendency that the concentration once decreases just after the administration and then turns to increase, in both of the deep and cortex portions. In cases of the sustaining administration, while, an increasing tendency was recognized after lapsed 10 minutes from the administration and in the groups of $5.0 \times 10^{-3}$ PNA units/kg or more administration.

Pharmacological Test Example 2

(1) As to possibility of cerebral edema generation

Normal or healthy rabbits were classified into following 3 groups.

Test group 1 :

Sustainingly administrated the kininogenase through vein at a dose of $2.5 \times 10^{-3}$ PNA units/kg.

Test group 2 :

Sustainingly administrated the kininogenase through vein at a dose of $5.0 \times 10^{-3}$ PNA units/kg.

Control group :

Sustainingly administrated saline.

Just after the administration, brain was exenterated to measure water content, $Na^+$, $K^+$ and $Cl^-$, and calculated a ratio of $Na^+/K^+$. There was found no significant difference between the both test groups and control group to judge that the administration of kininogenase shall not cause any cerebral edema.

(2) Oxygen intake into cerebral tissue and energy metabolism

Normal or healthy rabbits were classified into following 3 groups.

Test group 1 :

Sustainingly administrated the kininogenase through vein at a dose of $2.5 \times 10^{-3}$ PNA units/kg.

Test group 2 :

Sustainingly administrated the kininogenase through vein at a dose of $5.0 \times 10^{-3}$ PNA units/kg.

Control group :

Sustainingly administrated saline.

After lapsed 30, 60 and 90 minutes from the completion of the administration, brain was exenterated to measure intaking amount of oxygen as well as contents of ATP, pyruvate, lactate, glucose and glucose-6-phosphate to check an influences of the kininogenase to the intaking amount of oxygen and metabolisms.

It was found that the kininogenase causes to increase the intaking amount of oxygen in depending to an amount of its dose. There was found no significant difference in the contents of ATP, pyruvate, lactate, glucose and glucose-6-phosphate, between the test groups and control group.

Pharmacological Test Example 3

(Influence to model animal with hypoxia)

(1) Experimental method

Each of female New Zealand white rabbits (body weight of about 3kg) were anethetized with pentbarbital-Na, and a tube was inserted, at once, into their trachea to cause an adjusted breathing with air through a respirator.

After intravenously injecting 0.5mg/kg of tubocurarine chloride to cause an amyotonia, the animal was transferred to a fixing device to cause an orientation of her brain to peel bellies along a median line in its vertex and to form two apertures or windows of 0.5 x 0.5cm at right front portion and 1.0 x 0.5cm at right rear portion of the skull. To the front window, a silver ball electrode was set to measure brain-wave in the cortex, and a local blood flow in the cortex and $PO_2$ in brain were measured by utilizing the rear window. Number of heat beats was measured by making trigger R-wave of the brain-wave. These parameters were recorded by feeding the same to a polygraph, crossed thermocouple and recticorder.

After stabilized of each parameters, the air from the respirator was changed to 5% $O_2$ and at once, drugs given later was intravenously injected to continue the experiment for 10 minutes.

(2) Dose and dosing method of drugs

(A) Control group (n = 3) :
Administrated 3.3ml of phosphate buffer (0.05M, pH 7) for 30 minutes at rate of 0.11ml/min.
(B) 5.0 x $10^{-3}$ PNA units/kg Administration group (n = 3) :
Administrated 3.3ml of solution for 30 minutes at rate of 0.11ml/min., which solution was prepared by diluting a kininogenase solution of 5.0 x $10^{-2}$ PNA units/ml with phosphate buffer (0.05M, pH 7) by 10 folds.
(C) 2.5 x $10^{-3}$ PNA units/kg Administration group (n = 3) :
Administrated 3.3ml of solution for 30 minutes at rate of 0.11ml/min., which solution was prepared by diluting a kininogenase solution of 2.5 x $10^{-2}$ PNA units/ml with phosphate buffer (0.05M, pH 7) by 10 folds.
(D) 1.5 x $10^{-3}$ PNA units/kg Administration group (n = 3) :
Administrated 3.3ml of solution for 30 minutes at rate of 0.11ml/min., which solution was prepared by diluting a kininogenase solution of 1.5 x $10^{-2}$ PNA units/ml with phosphate buffer (0.05M, pH 7) by 10 folds.
(E) Citicholine (100mg/kg) administration group (n = 3) :
Administrated 3.3ml of a solution for 30 minutes at rate of 0.11ml/min., which solution was prepared by taking 4ml of Nicholine injection solution (250mg/2ml), and diluting the same with phosphate buffer (0.05M, pH 7) to make the drug concentration to 100mg/kg.
(F) Citicholine (10mg/kg) administration group (n = 3) :
Administrated 3.3ml of a solution for 30 minutes at rate of 0.11ml/min., which solution was prepared by taking 2ml of Nicholine injection solution (100mg/2ml), and diluting the same with phosphate buffer (0.05M, pH 7) to make the drug concentration to 10mg/kg.
(G) Nicardipine (4$\mu$ g/kg) administration group (n = 3) :
Taking 10mg of Nicardipine, and diluting the same with saline to make the drug concentration to 4$\mu$ g/kg to administrate 0.1ml/kg for 20 seconds.

(3) Method for processing data

A ratio of change was calculated on the number of heart beats, and an amount of change was calculated on the $PO_2$ in brain and local blood flow in brain. The brain-wave was analyzed with an analyzer (Type ATAC-450 marketed by Japan Photoelectric Co., Ltd.) to print out results. A statistic inspection or examination was carried out with use of a program of FM 11 marketed by Fujitu Co., Ltd. and judged as there is a significant difference, when P value is 0.05 or less in Student's t-test.

(4) Results

(A) Influence to number of heart beats

Although number of heart beats in the control group reduced with a significant difference, when it was compared with that in a none-treated control group, but there was no significant difference in the kininogenase and citicholine administration groups, when it was compared with that in the control group. While, in the Nicardipine administration group, number of heart beats reduced with a significant difference, when it was compared with that in the control group.

(B) Influence to $PO_2$ in cerebral cortex

An amount of $PO_2$ in cerebral cortex in the control group decreased with a significant difference, when it was compared with that in the none-treated control group. Namely, the control group showed a decrease of 8.1 x $10^{-8}$ PNA units/kg after lapsed 5 minutes from beginning of 5% $0_2$ gas sucking, but the none-treated control group did not show any decrease. On the contrary thereto, the kininogenase administration groups showed a decrease of 4.0 x $10^{-8}$ PNA units/kg at dose of 5.0 x $10^{-3}$ PNA units/kg and 3.0 x $10^{-8}$ PNA units/kg at dose of 2.5 x $10^{-3}$ PNA units/kg to find that the kininogenase significantly suppresses the decrease of $PO_2$ in cerebral cortex, which is caused in a state of low oxygen concentration.

In the groups administrated kininogenase at dose of 1.25 x $10^{-3}$ PNA units/kg as well as citicholine and Nicardipine, the decease is same level with that in the control group and no suppressing effect to decrease of $PO_2$ could be found. These results show that citicholine and Nicardipine as the control drugs do not show the effect for suppressing the decrease of oxygen amount in cerebral cortex, and that an effective amount of kininogenase is 2.5 x $10^{-3}$ PNA units/kg or more.

(C) Influence to local blood flow in cerebral cortex

An amount of local blood flow in cerebral cortex in the control group increased with a significant difference, when it was compared with that in the none-treated control group. Namely, the control group showed an increase of 26.5μ V after lapsed 5 minutes from beginning of 5% $0_2$ gas sucking, but the none-treated control group did not show any increase. On the contrary thereto, the kininogenase administration group (5.0 x $10^{-3}$ PNA units/kg) showed an increase of only 6.7μ V to find that this substance significantly suppresses the increase of the local blood flow in cerebral cortex.

It was no effect in the control drugs of citicholine and Nicardipine.

(D) Influence to brain-wave in cortex portion

In case of comparing the none-treated control group, a form of brain-wave in the control group showed a tendency of high-amplitude and slow wave. On the contrary thereto, a low-amplitude and quick wave was shown in the kininogenase administration groups at dose of 5.0 x $10^{-3}$ and 2.5 x $10^{-3}$ PNA units/kg as well as the citicholine administration group at dose of 100mg/kg. The results show that the kininogenase and citicholine changes the high-amplitude and slow wave due to sucking of air with low oxygen concentration to the low-amplitude and quick wave, by decreasing a slow/quick wave ratio, to improve the cerebral functions.

Pharmacological Test Example 4

Both of side vertebral arteries of a healthy rabbit were permanently ligated, both of the common carotid arteries were ligated for 40 minutes to make the brain in ischemic state, and then released the ligation of the common carotid arteries to observe a local blood flow and brain-wave in cerebral cortex portion and to measure concentrations of ATP and lactate in the cortex portion, at the time lapsed 15 minutes from the release of blood flow in the common carotid arteries.

Following drugs were administrated in following manner.

Kininogenase :

Intravenously administrated for 30 minutes from 15 minutes before the ligation of common carotid arteries and at a dose of 1.25 x $10^{-3}$, 2.5 x $10^{-3}$ or 5.0 x $10^{-3}$ PNA units/kg.

Citicholine :

Intravenously administrated for 30 minutes from 15 minutes before the ligation of common carotid

arteries and at a dose of 100mg/kg.

Nicardipine :

Rapidly administrated into an upper portion of small intestine at the time of 15 minutes before the ligation of common carotid arteries.

Release of blood flow in the common carotid arteries remarkably increased local blood flow in cerebral cortex portion. In this case, the Nicardipine showed a tendency to suppress the increase, but the kininogenase and citicholine did not suppresses the blood flow increase.

The brain-wave was flatten due to the ischemic, but reactivation thereof was recognized in the kininogenase administration group at dose of $2.5 \times 10^{-3}$ PNA units/kg. The reactivation was not recognized in the citicholine and Nicardipine administration groups.

The administration of the Nicardipine significantly increases ATP. The administration of the kininogenase at dose of $1.25 \times 10^{-3}$ PNA units/kg and Nicardipine significantly decreased the lactate. No significant difference was found on lipid peroxidase.

Results of this Example provides such an estimation that the kininogenase suppresses the increase of lactate to be formed by an anaerobic decomposition of saccharides due to ischemic, and activates flatten brain-wave.

Pharmacological Test Example 5
(Influence to cerebral circulation and metabolism in disease-model animal)

A micro-glass bead was injected into right hemicerebrum of an anethetized healthy rabbit to prepare a model animal with cerebral infarction. After administration of a drug, local blood flow (in cortex portion), micro-circulation in cerebral pia leptmeninges, distribution of cerebral blood flow and concentrations of $PO_2$, ATP, lactate, pyruvate, glucose and glucose-6-phosphate were measured. The measurement of biochemical substances was carried out after lapsed 60 minutes from the administration of the drug.

Results are shown below.

(1) Blood flow in cerebral cortex portion

In case of sustaining intravenous administration of the kininogenase at dose of $2.5 \times 10^{-3}$, $5.0 \times 10^{-3}$ or $1.25 \times 10^{-2}$ PNA units/kg, an increase of blood flow in cerebral cortex portion was recognized at ischemic side (glass bead injected side), but almost no increase was recognized in the other side. While, Nicardipine (0.5mg/kg) and citicholine (10mg/kg) as control drugs were administrated with rapid administration into upper small intestine and sustaining intravenous administration, respectively, but no increase in blood flow at the ischemic side was recognized.

(2) Micro-circulation in cerebral pia leptmeninges

A change in diameter arterioles in cerebral pia leptmeninges was observed, as to blood vessels having diameters of >100, >50 to <100 and <50$\mu$ m, under microscope and according to Cranial window method.

When the dose of kininogenase was set as $5 \times 10^{-3}$ PNA units/kg or less, apparent expansion of blood vessels with the diameter exceeding 50$\mu$ m can not be recognized, but more thin blood vessels concerning to a metabolism of substance apparently expanded. Apparently expansion was recognized also on the blood vessels with diameter exceeding 100$\mu$ m, when the dose thereof was increased to $1.25 \times 10^{-2}$ PNA units/kg.

On the contrary thereto, it was recognized on Nicardipine as one of control drug that it expand the blood vessels with diameter exceeding 100$\mu$ m, but almost no expansion was recognized on the blood vessels with diameter less than 50$\mu$ m.

The results show that the kininogenase effectively acts to thinner blood vessels and acting dynamics thereof are different from those of Nicardipine which is one of exemplar $Ca^+$-antagonistic drugs.

(3) Distribution of cerebral blood flow

A remarkable decrease in distribution rate of blood flow in gray matter or substance portion at the side of ischemic caused by the injection of glass bead, but the blood distribution was recovered to the level similar to none-injection side (normal side), by the administration of the kininogenase at the dose of $5 \times 10^{-3}$ PNA units/kg. On the contrary thereto, no improvement of blood flow distribution could be recognized by the administration of Nicardipine, as the control drug.

12

(4) Biochemical substances and others

It was found that the administration of kininogenase at the dose of $5 \times 10^{-3}$ PNA units/kg suppresses the decrease of $PO_2$, ATP, pyruvate, glucose and glucose-6-phosphate. The citicholine and Nicardipine did not show the suppress of ATP decrease.

Pharmacological Test Example 6
(Influence to region or area caused of cerebral infarction)

With use of model animals as in Pharmacological Test Example 4, an influence of a drug to region occurred a cerebral infarction at the time after lapsed 24 hours from the injection of glass bead was checked. Results are shown in following Table 5. As apparently seen therefrom, the kininogenase shows a significant effect, in comparison with a none-treated control and control drug.

Table 5

|  | Number of animal died/used (rate of die) | Region of infarction (%) | Inhibition (%) |
|---|---|---|---|
| Control | 5/11 (46%) | 22.7 ± 5.0 | - |
| Kininogenase (*) | 3/7 (43%) | 8.5 ± 6.0 | 62.6 |
| Citicholine (**) | 5/7 (71%) | 30.8 ± 19.8 | -35.9 |

In Table 5,
* : $2.5 \times 10^{-3}$ PNA units/kg, sustaining administration for 30 minutes, and
** : 100mg/kg, sustaining administration for 30 minutes.

Pharmacological Test Example 7
(Arousal reaction of brain-wave in cerebral cortex)

(1) Purpose

An influence of kininogenase to an improvement in an arousal reaction of brain wave in cerebral cortex was checked by selecting a rabbit as a test animal, electrically canterigating an internal capsule to prepare a model animal with symptoms similar to a person caused a cerebral hemorrhage, and administrating the drug, since the destruction of internal capsule gives a partial obstacle to an invigoration system in ascending rectacular region, which has been said as having a close relation with the arousal reaction of brain-wave in cortex.

(2) Operation method

(A) Preparation of a model animal destroyed in its internal capsule (Acute stadium model)

A cannula was fitted in an auricle vein of a female New Zealand white rabbit to anethetize the animal by intravenously administrating pentbarbital-Na (30mg/kg). The animal was fixed in the supine position to cut hairs at front neck and inguinal regions, and then a median incision was carried out to open a trachea for fitting therein a cannula at the neck portion, and to open a right femoral artery and vein at the inguinal region for fitting a cannula at the artery to detect a blood pressure to take a blood sample and at the vein to administrate a drug. Then, the animal was fixed in the prone position with use of a device for causing an orientation of her brain to cut hairs in head and incise head skin to open a skull. After intravenously injecting 3mg/body of tubocurarine chloride, the animal was subjected to adjusted breathing with room air through a respirator (15 - 30ml/body/stroke, and 30 - 40 strokes/min). A concentration of $PCO_2$ in the artery blood was periodically measured by a blood gas analyzer to adjust the respirator, so that the concentration lies in a suitable level of 35 ± 5mmHg).
In the next place, a two-pole adhered type electrode was sticked into the brain with reference to a brain chart, so that a free end thereof reach both internal capsules (APO, L : 6, H : O) and rectacular region (P : 8, L : 3, H : -2) of a left mesencephalon (mid-brain), to set the same with a dental cement. Among the electrodes, both of then reaching to the internal capsules are employed for cauterizingly destroy the internal capsules, and the other electrode reaching to the rectacular region of the mesencephalon is employed for stimulating a brain-wave. A screw type stainless steel electrode was set at a position (P : 3, L : 7) to measure the brain wave in cortex portion.

The brain-wave was monopolarly derived by the screw type electrode to record on a polygraph and also recorded on a magnetic tape by a data recorder. A cardiogram, blood pressure and number of heart beats were recorded on the polygraph through a secondary induction, a pressure transducer and a momentary heart beat recorder which subjects to cause a trigger the R wave for the cardiogram, respectively.

The destruction of internal capsules was carried out by passing 60V DC for 30 seconds, and then repeating the current passing with an interval of 5 minutes.

An observation of the brain-wave arousal reaction was done by high frequently stimulating the rectacular regions through the sticked electrode (rectangular wave fed through an electric stimulating device and isolator, frequency : 100Hz, voltage : 0.5 - 5.0V, interval : 1msec.) to measure changes of a threshold value and sustaining period of time on the arousal reaction.

After the measurements, the animal was killed by intravenously administrating saturated potassium chloride solution to exenterate the brain and reserved the same in 10% formalin solution to confirm the portions sticked the electrodes and regions caused obstacles, at later.

(B) Method for judging extent of arousal reaction

A voltage firstly appeared an arousal reaction, when a voltage for stimulating the rectacular regions shall be raised up with an interval of 5V, and a period of time during which the arousal reaction continues, when the rectacular regions are stimulated with voltage of 4V were defined as the "arousal reaction threshold value" and "arousal reaction sustaining time". The threshold value and sustaining time were visually judged from waves recorded on the charts and shown with units of voltage value (V) and time (sec.).

Brain-wave in the cortex for 2 minutes before the electric stimulation, in each time, was recorded in the data recorder, and the data was AD converted and Fourier converted by an data analyzer (Type ATAC-450 marketed by Japan Photoelectric Co., Ltd.) to check power spectrum. The analysis was carried out by dividing following frequency bands on power of 0.496 - 30Hz.

$\delta$ wave (0.496 - 3.968Hz),
$\theta$ wave (3.968 - 8.432Hz),
$\alpha$ wave (8.432 - 13.432Hz), and
$\beta$ wave (13.432 - 30Hz).

The power spectrum on each brain-wave was calculated as a ratio (%) a power in each frequency band at each time point to a total ECoG power.

(C) Measurement of local cerebral blood flow

A measurement of local cerebral blood flow was carried out by setting a free end of hydrogen electrode to peripheral portion (A : 4, L : 4, H : 3) of the internal capsule and the cerebral cortex portion and utilizing a hydrogen clearance method. The measurement according to the hydrogen clearance method was carried out by a tissue blood flow meter (Type RBF-2 marketed by Biomedical Co., Ltd.) and with hydrogen inhalation system. A calculation of local cerebral blood flow was carried out with an aid of a computer by feeding data from the tissue blood flow meter through an A/D converter, or reading out the chart from the blood flow meter to feed the same to the computer. The measurement of local cerebral blood flow was done only on control group, and a brain wave action was not measured.

(D) Method for confirming impediment in blood-brain barrier

To confirm whether an impediment caused in a blood-brain (BBB) or not, Evans's blue solution was intravenously injected just after the destroy of internal capsules and then exenterated the brain after lapsed 1 hour from the injection. This experiment was carried out only for the control group.

(E) Method for processing data

The threshold value and sustaining time in the arousal reaction, $\delta$ , $\theta$ , $\alpha$ and $\beta$ waves as well as local cerebral blood flow were obtained with units of V, sec., % and ml/min/100g, and number of heat beats and mean blood pressure were obtained with a charging ratio (%) to the values to that before the destruction of internal capsules.

Each of the differences on the parameters between the control group and other groups was examined with the Student's t-Test, and judged as that there is a statistic significant difference, when P value is less than 5%.

(F) Dose

(a) Control group (n = 7) :
Intravenously administrated for 30 minutes phosphate buffer (0.05M, pH 7) at a rate of 0.11ml/min.
(b) False-operation group (n = 6) :
(c) $1.25 \times 10^{-2}$ PNA units/kg Administration group (n = 7)
Intravenously administrated 3.3ml of solution for 30 minutes at a rate of 0.11ml/min, which solution was prepared by diluting a kininogenase solution of $1.25 \times 10^{-1}$ PNA units/ml with phosphate buffer (0.05M, pH 7) by 10 folds.
(d) $5.0 \times 10^{-3}$ PNA units/kg Administration group (n = 7) :
Intravenously administrated 3.3ml of solution for 30 minutes at a rate of 0.11ml/min, which solution was prepared by diluting a kininogenase solution of $5.0 \times 10^{-2}$ PNA units/ml with phosphate buffer (0.05M, pH 7) by 10 folds.
(e) Citicholine (100mg/kg) administration group (n = 6) :
Intravenously administrated 3.3ml of a solution for 30 minutes at a rate of 0.11ml/min, which solution was prepared by taking out 4ml of Nicholine injection solution (250mg/2ml), and diluting the same with phosphate buffer (0.05M, pH 7) to make the drug concentration to 100mg/kg.

(3) Results

(A) Influence to arousal action of cortex brain-wave

(a) Threshold value of arousal reaction

Comparing with the control group, a threshold value in the false-operation group showed a tendency of increase, but it was not recognized as a significant change. No apparent change was recognized in the kininogenase and citicholine administration groups, and no significant difference was found, in comparison with the value in the control group.

(b) Sustaining period of time in the arousal reaction

A sustaining period of time in the arousal reaction in the control group was significantly increased in comparison with that in the false-operation group. Comparing to the control group, the sustaining period of time in the kininogenase administration group at the dose of $1.25 \times 10^{-2}$ PNA units/kg was significantly increased at the time lapsed 60 minutes from the internal capsule destruction, namely at the time just finished the sustaining administration of the drug, and same tendency was recognized at other time points. A significant extension or shortage on the sustaining period of time was not recognized in the kininogenase administration group at dose of $5.0 \times 10^{-3}$ PNA units/kg and citicholine administration group.

(B) Action to each frequency component of brain wave in cortex portion

It was recognized that the brain wave in cortex portion on the control group for 2 minutes before the starting of arousal reaction due to electrical stimulation to the rectacular region in mid-brain apparently slow-down, in comparison with that in the false-operation group. A rate of the $\delta$ wave occupying in the total power increased just after the internal capsule destruction, but on contrary thereto, the $\theta$ wave decreased. Such a tendency was recognized that the $\alpha$ and $\beta$ waves as quick wave components decrease due to the internal capsule destruction.

Such a tendency was recognized in the groups administrating kininogenase and citicholine that the reduction of quick wave components is suppressed.

15

(C) Actions to local cerebral blood flow, number of heart beats, and mean blood pressure

(a) Local cerebral blood flow

A local cerebral blood flow in acute stadium of the internal capsule destruction significantly decreased in peripheral portions of the destroyed internal capsule, but almost no influence was recognized to the blood flow in the cerebral cortex.

(b) Number of heart beats

No significant difference was recognized on the number of heart beats between the control and false-operation groups. A transitional decrease depending on an amount of dose was recognized in the kininogenase administration groups. The number of heart beats in the citicholine administration group decreased more than that in the kininogenase administration groups.

(c) Mean blood pressure

No significant difference was recognized on the mean blood pressure between the control and false-operation groups. No significant difference was also recognized between the control group and citicholine administration group, but in the kininogenase administration group at high dose ($1.25 \times 10^{-2}$ PNA units/kg), a significant difference was recognized at the time lapsed 15 minutes from the starting of its administration.

(D) Action to blood-brain barrier

A leakage of Evans's blue was recognized at the blood-brain barrier in acute stadium of the internal capsule destruction, but almost no leakage was found in the false-operation group, to which an electrode was sticked but internal capsule was not destroyed.

Pharmacological Test Example 8
(Action to nerve dysfunction, cerebral circulation and brain wave to disease model animal)

A tube was fitted to left-outernal carotid artery of anesthetized Japanese monkey and a silicone resin pellet [1mm ($\phi$) x 5mm] was inserted into the artery through the tube to prepare a middle cerebral artery obstructive model animal.

After lapsed 5 minutes from the obstruction of left middle cerebral artery, kininogenase ($2.5 \times 10^{-2}$ PNA units/kg) or citicholine (10mg/kg) as a test and control drugs was administrated through an intravenous infusion.

After lapsed 24 hours from the obstruction of the left middle cerebral artery due to silicon pellet, An apparent nerve symptoms including a hemiplegia due to dysfunction in left cerebral hemisphere were recognized in a control group given no drug. No apparent difference was recognized in blood flow of cortex portion in left hemisphere (no dysfunction side), in comparison with a false-operation group, but the blood flow in the left hemisphere reduced to about 1/3. Further, the $\alpha$ and $\beta$ waves as quick wave components of cerebral activity in left and right front, top and side areas reduced to 20 - 30%, in comparison with those in the false-operation group, to show an apparent state of cerebral dysfunction.

On the contrary thereto, almost no nerve dysfunction can be recognized in the kininogenase administration group, a blood flow in cerebral cortex portion showed a lowering tendency in the right hemisphere (no dysfunction side), but an increasing tendency was observed in left hemisphere of dysfunction side, in comparison with the control group, and a ratio of blood flows between the left and right hemispheres was about 1. As to brain-wave activities, an increasing tendency was recognized in rapid wave components, excepting right top area, and the tendency was remarkable in left front area to be considered as a border area in ischemic.

While, the group administrated citicholine as the control drug showed an improvement in the neuro symptoms, in comparison with the control group, but no improvement was recognized on blood flow in cerebral cortex and brain wave activities, so that it can not be said that this drug is effective for improving cerebral functions.

Pharmacological Test Example 9 (Test for confirming safety)

To healthy male persons, single administration tests on the kininogenase by a dose of 0.01, 0.02, 0.04, 0.75, 0.15 or 0.30 PNA units/body and repeating administration test over 3 days at dose of 0.15 PNA units/body/day were carried out.

Tested persons were 3 individuals for each group (Total : 21 persons), whom age, height and body weight were 21 - 27 years old, 160 - 183cm and 51 - 78kg, respectively. The test compound was dissolved by a predetermined amount into 100ml of 5% grape sugar solution according to the Japanese Pharmacopoeia and administrated by an intravenous drip infusion over 30 minutes from great saphenous vein.

A subjective symptom, blood pressure, number of heart beats, body temperature, number of breath, and steoscopy were observed as crinical symptom observation items for evaluation. As crinical examinations, hematological examinations, serum biochemical examinations, examinations on dissolution-agglomeration system in gland, urine examinations, antibody examination (agglomeration test using sensitized erythocites), electrocardiogram and examination of behavior in blood plasma were carried out.

In observations of crinical symptoms, a flushing in face which shall be estimated by somewhat action due to the kininogenase was recognized for 30 minutes, on 2 persons within 3 one in maximum amount of 0.30 PNA units/body administration group for the single administration test. Further, a reducing tendency in diastolic pressure was recognized during the intravenous infusion period of time, in 0.15 and 0.30 PNA units/body administration groups for the single administration test and the repeated administration group (over 3 days of 0.15 PNA/body/day), and an increasing tendency was recognized in number of heart beats on the groups administered 0.075 PNA units/body or more, but other abnormal state to be estimated as causing by the kininogenase can not be recognized.

On crinical examinations, any change in value, to be estimated by the kininogenase, was not recognized. No production of antibody to the kininogenase was found in the examination of antibody.

The kininogenase was not detected in plasma in 0.01 and 0.02 PNA units/body administration groups, but detected in 0.04 PNA units/body and more administration groups. A good linear relation was recognized between each value of $C_{30min}$ and $AUC_{0-24}$ in 0.04, 0.075, 0.15 and 0.30 PNA units/body administration groups and the amount of dose. A value of $t_{1/24}$ was about 170 minutes.

In the repeated administration test, a similar transition or change of kininogenase concentration in plasma showed in both test period in the first and third day, and there was almost no difference in drug dynamic parameters, and thus it had been considered that an accumulation of the drug in body shall not occur, even if the kininogenase shall be continuously administrated.

Pharmacological Test Example 10
(Test for setting effective dose)

To in-patients with symptoms due to cerebral infarctions (cerebral thrombosis, cerebral embolism and others), lapsed 1 month from their paroxysm and judged as a chronic state, kininogenase was administrated in following manner for 3 weeks, by classifying to sustaining intravenously administration groups for 30 minutes (28 persons) and for 120 minutes (14 persons).
1st week : 0.075 PNA units/body/day,
2nd week : 0.15 PNA units/body/day, and
3rd week : 0.30 PNA units/body/day.

In the group administrated for 30 minutes, abandonment of the administration occurred at 5th day of the first week by two persons, one of them with an urticarial exanthemia and face flushing, and the other with a palpitation, shakes and diarrhea, in 3rd week by 5 persons, among them 1 person with raising of blood pressure and flush feeling, 2 persons with face flushing, 1 person with burning of face, and the other person with face flushing, and thus certain side-effects generated to 7/28 persons (25%).

While, in the other group administrated for 120 minutes, abandonment of the administration occurred at 6th day of the 3rd week by one person with a diarrhea which occurred in the first week, and another person with a hot feel in head. Therefore, a generation of side-effect was 2/14 persons (16.7%).

An effectiveness was not apparent in the first week, an improvement of symptoms was recognized during the second week, but a remarkable improvement was not found in the third week. Therefore, it may be so estimated that a difference in pharmacological effects between the doses of 0.15 and 0.30 PNA units/body is small.

By taking the above facts and other facts of that there is no remarkable difference in safety between the groups administrated for 30 and 120 minutes and that the generation of side effect concentrates to the time in the third week administrated the drug at maximum dose of 0.30 PNA units/body into consideration, it can be judged that an optimum dose and administration method of kininogenase are 0.15 PNA units/body and sustaining intravenous influsion for 30 minutes.

Pharmacological Test Example 11 (Crinical tests)

Deputing to various institutions in Japan, crinical tests of kininogenase were carried out as subjects of in-patients with symptoms due to cerebral infarctions (cerebral thrombosis, embolism and the like) or cerebral hemorrhage, lapsed 1 month from their paroxysm and judged as a chronic state.

(1) Testing method

(A) Testing drugs

(a) Placebo group :
Placebo (1.5ml/body/day).
(b) Low dose group :
0.5ml of Kininogenase (0.075 units) + placebo (1.0ml)/body/day.
(c) High dose group :
0.5ml of Kininogenase (0.150 units) + placebo (1.0ml)/body/day.

(B) Method of dose and dosing period of time

The drug was added to a supplemental solution (200ml) to intravenous drip administrate the same for 30 minutes. A period of time for the administration was 2 weeks.

(C) Drug using jointly

As a rule, drugs of a cerebral vasodilator, cerebral metabolism invigorator and the like which may give an influence of the test drug were not administrated.
When it necessary to give another drug, avan may be given by 1 tablet for once and 3 tablets/day, provided that the drug as one of the cerebral metabolism invigorators had been given for 1 months or more before the time for starting this test.

(D) Method of analysis

Measured data was processed by a t-examination method (comparison in a group), a dispersion analysis (comparison between groups) and Scheffe type multi-comparison method, order data was treated by H-examination method by Kruskal-Wallis (hereinafter referred to as "H-examination) and Scheffe type multi-comparison method, and classification data was processed by $\chi^2$-examination method. Standard for judging a significance was set as 5%, but 10% was also considered as a Reference.

(2) Persons to be tested

(A) Subjects

Total number of patient at starting time was 306 persons, but among them 3 persons were excluded due to leaving from the hospital, not so lapsed in time from paroxysm, or a generation of a complication, respectively, and thus 303 persons were tested. However, it includes 35 persons who can not be said as the suitable subject in a strict meaning due to an offence on a jointly administrated drug, stopping of administration by generation of side-effect and the like, but data thereon were partly employed, namely 8 individuals in the placebo administration group (hereinafter referred to as "P group"), 17 individuals in the kininogenase administration group of low dose (hereinafter referred to as "L group"), and 10 individuals in the kininogenase administration group of high dose (hereinafter referred to as "H group").

(B) Background factors of subjects

Background factors were searched and analyzed to obtain results shown in Table 6 (Tables 6 - 1 and 6 - 2). As apparently seen therefrom, no significant difference was recognized.

### Table 6 - 1

| I t e m | Total | Group | | | Result |
|---|---|---|---|---|---|
| | | P | L | H | (Method of Examination) |
| | 303 | 101 | 103 | 97 | |
| Sex distinction | | | | | NS |
| Male | 194 | 65 | 62 | 67 | |
| Female | 109 | 36 | 41 | 32 | (χ2) |
| Age | | | | | |
| 30 - 39 | 1 | 0 | 1 | 0 | |
| 40 - 49 | 10 | 3 | 2 | 5 | NS |
| 50 - 59 | 61 | 23 | 19 | 19 | |
| 60 - 69 | 91 | 28 | 33 | 30 | (H) |
| 70 - 79 | 114 | 42 | 35 | 37 | |
| 80 - 89 | 26 | 5 | 13 | 8 | |
| Diagnosed disease | | | | | NS |
| Cerebral infarction | 247 | 82 | 86 | 79 | |
| Cerebral hemorrhage | 56 | 19 | 17 | 20 | (χ2) |
| Position of focus (*) | | | | | |
| Internal carotid artery | 226 | 73 | 77 | 76 | NS |
| Vertebral artery | 36 | 12 | 14 | 10 | |
| Both of the above | 31 | 11 | 11 | 9 | (χ2) |
| Unidentified | 9 | 4 | 1 | 4 | |
| Symptom | | | | | |
| Heavy | 59 | 24 | 22 | 13 | NS |
| Middle | 190 | 61 | 62 | 67 | |
| Light | 54 | 16 | 19 | 19 | (H) |

Table 6 - 2

| | | | | | |
|---|---|---|---|---|---|
| Contraction period of time | | | | | |
| < 3 months | 186 | 68 | 58 | 60 | NS |
| 3 - 6 months | 50 | 14 | 21 | 15 | |
| > 6 months | 67 | 19 | 24 | 24 | (H) |
| Incipient or recrudescence | | | | | |
| Incipient | 231 | 79 | 76 | 76 | NS |
| Recrudescence | 72 | 22 | 27 | 23 | ($\chi$2) |
| Complication | | | | | |
| No | 44 | 15 | 14 | 15 | NS |
| Yes | 259 | 86 | 89 | 84 | ($\chi$2) |
| Drug preused | | | | | |
| No | 94 | 26 | 34 | 34 | NS |
| Yes | 209 | 75 | 69 | 65 | ($\chi$2) |
| Drug used jointly (**) | | | | | |
| No | 222 | 71 | 76 | 75 | NS |
| Yes | 81 | 30 | 27 | 24 | ($\chi$2) |
| Avan used jointly | | | | | |
| No | 260 | 81 | 92 | 87 | NS |
| Yes | 43 | 20 | 11 | 12 | ($\chi$2) |
| Rehabilitation | | | | | |
| No | 99 | 33 | 32 | 34 | NS |
| Yes | 204 | 68 | 71 | 65 | ($\chi$2) |

In Table 6 (Tables 6 - 1 and 6 - 2),

* : 1 individual was excluded from P group (administrated placebo only), since CT-scanning has not yet been carried out,

** : Use a cerebral vasodilator, cerebral metabolism invigorator including avan, and

NS : P > 0.10

(3) Test results (Total evaluation)

(A) General improvement

General improvements in the first week was 1.1% in P group, 7.9% in L group and 5.4% in H group, in the level of middle or more improvement, and it increased to 44.2% in P group, 55.1% in L group and 55.9% in H group, when a case of light improvement was included. Results of H-examination showed that there is no significant difference in the groups.

General improvements in the second week was 12.9% in P group, 23.3% in L group and 28.1% in H group, in the level of middle or more improvement, and it increased to 65.6% in P group, 75.6% in L group and 83.1% in H group, when a case of light improvement was included. Results of H-examination showed that there is a significant difference in the groups (P < 0.01). A multi-comparison showed that H group is significantly excellent in comparison with P group (P < 0.01).

(B) Safety

A percentage of that in the first week, there is no problem in safety was 92.1% in P group, 88.3% in L group and 90.1% in H group. In the second week, the value was changed to 94.8% in P group, 88.3% in L group and 89.1% in H group. Final judgement showed 91.1% in P group, 82.5% in L group and 86.9% in H group. Results of H-examination showed that there is no significant difference in the groups in all evaluation time point.

(C) Usefulness

A usefulness in the first week was 2.0% in P group, 9.5% in L group and 7.3% in H group, in the level of fair or more usefulness, and it increased to 39.8% in P group, 50.5% in L group and 55.2% in H group, when a case of light improvement was included. Results of H-examination showed that there is a certain significant difference in the groups (P < 0.10). A multi-comparison showed that H group is excellent in comparison with P group (P < 0.10).

The usefulness in the second week was 12.9% in P group, 21.8% in L group and 30.3% in H group, in the level of fair or more usefulness, and it increased to 61.3% in P group, 70.1% in L group and 83.1% in H group, when a case of light improvement was included. Results of H-examination showed that there is a significant difference in the groups (P < 0.001). A multi-comparison showed that H group is significantly excellent in comparison with P group (P < 0.001).

(4) General improvement classified into indications

(A) Subjective symptom

An improvement in subjective symptoms in the second week was 13.0% in P group, 21.9% in L group and 21.2% in H group, in the level of middle or more improvement, and it increased to 58.0% in P group, 62.5% in L group and 84.8% in H group, when a case of light improvement was included. Results of H-examination showed that there is a significant difference in the groups (P < 0.01). A multi-comparison showed that H group is significantly excellent in comparison with P group (P < 0.05).

(B) Mental indication

An improvement in mental indications in the second week was 11.8% in P group, 17.1% in L group and 21.8% in H group, in the level of middle or more improvement, and it increased to 55.3% in P group, 64.5% in L group and 75.6% in H group, when a case of light improvement was included. Results of H-examination showed that there is a significant difference in the groups (P < 0.05). A multi-comparison showed that H group is significantly excellent in comparison with P group (P < 0.05).

(C) Nerve indication

An improvement in nerve indications in the second week was 2.2% in P group, 7.0% in L group and 9.0% in H group, in the level of middle or more improvement, and it increased to 40.9% in P group, 40.7% in L group and 56.2% in H group, when a case of light improvement was included. Results of H-examination showed that there is a significant difference in the groups (P < 0.05). A multi-comparison showed that H group is excellent in comparison with P group (P < 0.10).

(D) Behavior in daily activity

An improvement on behavior in daily activities in the second week was 8.0% in P group, 8.5% in L group and 9.5% in H group, in the level of middle or more improvement, and it increased to 40.9% in P group, 48.8% in L group and 54.8% in H group, when a case of light improvement was included. Results of H-examination showed that there is no significant difference in the groups.

(5) Improvement classified into items of indication

(A) Subjective symptom

Results of H-examination showed that there is no significant difference in the groups on improvement classified into each item. Symptoms recognized more than 10 individuals and showed a relatively high improvement (20% or more in the level of middle or more improvement) were a rotary vertigo (30.0%), stiffness in the shoulder and neck (25.0%), tennitus (23.1%), cephalagia (21.7%) and vertigo (21.7%), all of those being recognized in H group,

(B) Mental indication

Results of H-examination on improvement in each item showed that there are difference in the groups on a affective disorder or emontinal disturbance, and retention defect (P <0.10). Multi-comparison showed that the H group is excellent than L group in both items. Symptoms recognized more than 10 individuals and showed a relatively high improvement (20% or more in the level of middle or more improvement) were a depression (22.2%), and anxiety • impatience (20.0%), these being recognized in H group.

(C) Nerve indication

Results of H-examination on improvement in each item showed that there is a significant difference in the groups on a dysphagia (P < 0.05). Multi-Comparison showed that the H group is significantly different from the L group (P < 0.05).

(D) Behavior in daily activity

Results of H-examination on improvement in each item showed that there is a significant difference in the groups on a dysbasia (P < 0.05). Multi-Comparison showed that the H group is significantly different from the L group (P < 0.05).

(6) Improvement classified into background factors

On following background factors, a significant difference or a tendency thereof was recognized in the groups through H-examination and multi-comparison, all of those being recognized in H group.

Male in Sex distinction; cerebral infarction in Diagnosed disease; internal carotid artery in Position of focus; heavy and light in Symptom; 3 - 6 months and more than 6 months in Contraction period of time; incipient and recrudescence; yes in Complication; yes in Drug preused; no in Drug used jointly; no in Avan used jointly; and yes in Rehabilitation.

(7) Side affect

Generation of side effects was 10 individuals (9.9%) in P group, 19 individuals (18.4%) in L group, and 12 individuals in H group, and there is recognized no significant difference in the groups.

The most occasional side-effect was a diarrhea which was recognized in 7 individuals (1 in P group, 4 in L group, and 2 in H group). Subsequent side-effects are as follows.

a) Descending in blood pressure :

6 individuals (1 in P group, 2 in L group, and 3 in H group),

b) Involuntary motion or movement :

3 individuals (1 in each group),

c) Vertigo :

3 individuals (1 in P group, and 2 in H group),

d) Nausea :

3 individuals (2 in L group, and 1 in H group).

Among them, an extent of side-effects was evaluated as "heavy" on 3 dividuals (2 in L group, and 1 in H group).

(8) Blood pressure and number of heart beats

Comparison in group (t-examination with correspondence) treated a value obtained before administration and values in the first and second week showed a significant reduction in H group on a systolic pressure in the first week (P < 0.05). Results of multi-examination showed a similar tendency between L and H groups (P < 0.10). A tendency of significant difference was found on a diastolic pressure in the second week (P < 0.10), but multi-examination showed that there is no significant difference. A tendency of significant difference was found in number of heart beats in the first week (P < 0.10), and results of multi-examination showed that there is such a tendency between L and H groups.

(9) Crinical examination

Comparison in group (t-examination with correspondence) treated values obtained before and after administration showed a tendency of significant difference on some items, but all of changes is in minor and no significant difference was recognized in any item important from crinical point of view.

Pharmacological Test Example 12
(Test for setting effective dose)

To patients as subjects with an ulcer in limbs (light - middle symptoms) due to a thromboangilis obliterans or arteriosclerosis obliterans and confirmed a position of infarct in blood vessels, kininogenase (ampuled product obtained by Example 1) was diluted with a supplemental solution, and administrated in following manner, through sustaining intravenous influsion for 30 minutes, for 2 weeks test period of time.

Group A :      0.075 PNA (units/body/day),

B :              0.15,

C :              0.30.

Diameter of the ulcer was periodically measured to calculate mean diameter (mm) and reduction ratio ($\sqrt{}$ major axis x $\sqrt{}$ minor axis) to obtain results shown in following Tables 7 and 8. As apparently seen therefrom, an effectiveness can be recognized in the groups at dose of 0.15 - 0.30 PNA units/body. A reduction in mean diameter of the ulcer was recognized in the group at dose of 0.30 PNA units/body, but in the reduction ratio, the group at dose of 0.15 PNA units/body is excellent and thus it shall be said the latter dose is preferable for avoiding a possible generation of side-effect.

Table 7

| Group | Initiation | After 1 week | After 2 weeks |
|-------|-----------|--------------|---------------|
| A | 16.3 ± 5.5 (10) | 14.9 ± 6.4 * (08) | 13.6 ± 5.2 (09) |
| B | 16.0 ± 4.2 (08) | 13.0 ± 5.2 * (07) | 11.2 ± 4.8 ** (08) |
| C | 10.6 ± 1.9 (09) | 8.0 ± 1.7 * (08) | 6.6 ± 1.1 ** (09) |
| In Tables 7 and 8, Value : mean ± S.E. (mm), ( ) : Number of patients, | | | |
| * : P > 0.05, and ** : P > 0.01. | | | |

23

Table 8

| Group | After 1 week | After 2 weeks |
|---|---|---|
| A | 21.2 ± 7.9 * (08) | 27.8 ± 15.3 (09) |
| B | 32.4 ± 13.2 * (07) | 49.0 ± 13.4 ** (08) |
| C | 23.5 ± 7.2 * (08) | 35.1 ± 7.3 ** (09) |
| In Tables 7 and 8, Value : mean ± S.E. (mm), ( ) : Number of patients, | | |

\* : P > 0.05, and
\*\* : P > 0.01.

Pharmacological Test Example 13 (Crinical test)

Deputing various institutions in Japan, crinical tests of kininogenase were carried out as subjects who are patients with arterioscleosis obliterans or thromboangilis obliterans, with an ulcer in limbs, a position of infarct in blood vessel having been confirmed.

(1) Testing method

(A) Testing groups

(a) Low dose group :
0.5ml of kininogenase (0.075 PNA units) + Placebo (1.0ml)/body/day, and
(b) High dose group :
0.5ml of kininogenase (0.150 PNA units) + Placebo (1.0ml)/body/day.

(B) Method of dose and dosing period of time

Ampuled product obtained by Example 3 was diluted into 200ml of supplemental solution and intravenous drip administrated for 30 minutes. The testing period of time was 2 weeks.
Prior to initiation of the test, 1 week observation period of time was provided to check drugs which should not be jointly used.

(C) Method of analysis

Measured data was processed by a t-examination method, order data by Mann-Whitney's U-examination method, and classification data by $\chi^2$-examination method and Fisher's direct probability calculation method. Standard for judging a significance was set as 5%, but 10% was also considered as a Reference.

(2) Persons to be tested

(A) Subjects

Total number of patients at starting time was 84 persons [40 individuals in low dose group (hereinafter referred to as "L group" and 44 individuals in high dose group (hereinafter referred to "H group")], but among them, 3 individuals in H group were excluded due to an offence on jointly administrated drug (1 individual) and chronic renal insufficiency (2 individuals), and thus 81 persons were tested. However, it includes 35 persons who can not be said as suitable subjects in a strict meaning due to an offence of jointly used drug, stopping of the test drug administration by a generation of side-effect and the like, but data thereon were partly employed, namely 16 individuals in L group and 19 individuals in L group.

(B) Background factors of subjects

Among 81 persons as the subjects for analysis, 66 individuals concerned to usefulness, so that background factors of the patients were searched and analyzed to obtain results shown in following Table 9 (Tables 9 - 1 to 9 - 3). As apparently seen from the Table, no significant difference was recognized in any Item.

## Table 9 - 1

| I t e m | L group | H group | Result (Exami-nation method) |
|---|---|---|---|
| Sex distinction : | | | |
| Male | 23 | 29 | NS |
| Female | 6 | 8 | (χ 2) |
| Age : | | | |
| < 39 | 1 | 6 | NS (U-exam.) |
| 40 - 59 | 13 | 16 | ditto |
| 60 - 79 | 12 | 13 | ditto |
| > 80 | 3 | 2 | ditto |
| Mean ± SE | 61.6 ± 2.6 | 57.0 ± 2.8 | NS (t-exam.) |
| Classification in treating : | | | |
| In-patient | 23 | 32 | NS |
| Out-patient | 6 | 5 | (χ 2) |
| Diagnosed disease : | | | |
| Thromboangilis obliterans | 14 | 22 | NS |
| Arterioscleosis obliterans | 15 | 15 | (χ 2) |
| Contraction period of time : | | | |
| < 3 Months | 20 | 17 | NS |
| 3 - 12 Months | 7 | 18 | (χ 2) |
| > 12 Months | 2 | 2 | P < 0.1 |
| Mean ± SE | 3.9 ± 1.4 | 4.9 ± 1.7 | NS (t-exam.) |
| Symptom : | | | |
| Light | 9 | 9 | NS |
| Middle | 14 | 18 | |
| Heavy | 6 | 10 | (U-exam.) |

25

## Table 9 - 2

| Infarcted position : | | | |
|---|---|---|---|
| Upper limb(s) | 4 | 3 | NS |
| Lower limb(s) | 25 | 34 | (χ 2) |
| Position of ulcer : | | | |
| Upper limb(s) | 4 | 3 | NS |
| Lower limb(s) | 25 | 34 | (χ 2) |
| Record of operations : | | | |
| No | 16 | 23 | NS |
| Yes | 13 | 14 | (χ 2) |
| Accompanying disease : | | | |
| No | 20 | 24 | NS |
| Yes | 9 | 13 | (χ 2) |
| Anamnesis : | | | |
| No | 17 | 23 | NS |
| Yes | 12 | 14 | (χ 2) |
| Drug used jointly : | | | |
| No | 19 | 24 | NS |
| Yes | 10 | 13 | (χ 2) |
| Size of ulcer (*) : | | | |
| < 10mm | 12 | 16 | NS (U-exam.) |
| > 10 - < 20mm | 11 | 16 | ditto |
| > 20mm | 6 | 5 | ditto |
| Mean ± SE | 14.5 ± 2.5 | 15.1 ± 3.0 | NS (t-exam.) |

Table 9 - 3

| Condition of granulation : | | | |
|---|---|---|---|
| Cured | 0 | 0 | |
| Benignity | 2 | 3 | NS |
| Somewhat benignity | 5 | 11 | |
| Somewhat malignancy | 13 | 14 | (U-exam.) |
| Malignancy | 9 | 9 | |
| Infection : | | | |
| No | 19 | 24 | NS |
| Yes | 10 | 13 | (χ 2) |
| Algia in rest time : | | | |
| No pain | 1 | 1 | |
| Pain in unusual | 3 | 7 | NS |
| Occasionally require analgesic | 9 | 16 | (χ 2) |
| Always require analgesic | 9 | 9 | P < 0.05 |
| Strong pain disturbing sleep | 7 | 2 | |
| Shake in limbs | | | |
| No shake feeling | 6 | 8 | |
| Very light | 1 | 6 | NS |
| Light | 5 | 8 | (U-exam.) |
| Middle | 11 | 12 | |
| Heavy | 6 | 3 | |

In Table 9 (Tables 9 - 1 to 9 - 3),

  &ast; : √ major axis x √ minor axis

(3) Test results (Total evaluation)

(A) General improvement

General improvements in the first week was 16.7% (4/24) in L group and 22.2% (6/27) in H group, in the level of middle or more improvement, and it increased to 45.8% (11/24) in L group and 77.8% (21/27) in H group, when a case of light improvement was included. H group showed a significant difference in comparison with L group (P < 0.05).

General improvements in the second week was 48.3% (14/29) in L group and 48.6% (17/35) in H group, in the level of middle or more improvement, to show no significant difference therebetween, but it changed to 58.6% (17/29) in L group and 94.3% (33/35) in H group to show a significant difference (P < 0.01), when a case of light improvement was included.

(B) Safety

A percentage of that in the first week, there is no problem in safety was 91.9% (34/37) in L group and 93.0% (40/43) in H group. In the second week, the value changed to 87.5% (28/32) in L group and 93.0% (41/43) in H group, but there was recognized no significant difference therebetween in both evaluation time points.

(C) Usefulness

A usefulness was searched using the value of "mean ± SE" to know that H group shows an excellent value of 75.78 ± 3.26 in comparison with that of 66.47 ± 4.83 in L group, but there was recognized no significant difference.
The usefulness was evaluated by classifying following items.

| | |
|---|---|
| a) Very useful | 100 - 91, |
| b) Useful | 90 - 71, |
| c) Somewhat useful | 70 - 51, |
| d) Can not be said as useful | 50 - 21, and |
| e) Not preferable | 20 - 0. |

The usefulness was 55.2% (16/29) in L group and 62.2% (23/37) in H group, in the level of "Useful" or more and thus no significant difference was recognized between the groups, but it changed to 62.1% (18/29) in L group and 89.2% (33/37) in H group to find a significant difference (P < 0.05), when the judging standard was level downed to "somewhat useful".

(4) Evaluation in each item

(A) An improvement in the first week was 46.4% (13/28) in L group and 60.0% (21/35) in H group, in the level "reduction" or more, and there was recognized no significant difference between the groups, but it increased to 57.1% (16/28) in L group and 82.9% (29/35) in H group to find a significant difference (P < 0.05), when the judging standard was level downed to "somewhat reduction".
(B) Change in size of ulcer
A size of ulcer in L group was reduced in both of the first and second weeks, in comparison with that before the administration, but there was found no significant difference. On the contrary thereto, a size of ulcer in H group was reduced to 84.29 ± 4.53% in the first week and 64.20 ± 6.55% in the second week, when size of ulcer before the administration was set as 100% to show significant differences (P < 0.01 and P < 0.001, respectively).
(C) Condition of granulation
Changes in condition of granulation are shown in following Table 10 and there was found no significant difference between the groups, but improvements in L group were 20.8% (5/25) in the first week and 39.3% (11/28) in the second week, in the level of middle" or more. While, in H group, those were 29.6% (8/24) in the first week and 40.0% (14/35) in the second week. When the judging standard was level downed to "light" or more, percentages were increased to 50.0% (12/24) in the first week and 64.3% (18/28) in the second week for L group, and 59.3% (16/27) in the first week and 80.0% (28/35) in the second week for H group to find out that the kininogenase has an action for improving the condition of granulation.

Table 10

| Item | After 1 week | | After 2 weeks | |
|---|---|---|---|---|
| | L group | H group | L group | H group |
| Remarkably improved | 0 ( 0) | 2 ( 7.4) | 8 (28.6) | 5 (14.3) |
| Improved in middle | 5 (20.8) | 6 (22.2) | 3 (10.7) | 9 (25.7) |
| Improved in light | 7 (29.2) | 8 (29.6) | 7 (25.0) | 14 (40.0) |
| Not changed | 9 (37.5) | 11 (40.7) | 5 (17.9) | 6 (17.1) |
| Aggravated | 3 (12.5) | 0 ( 0) | 5 (17.9) | 1 ( 2.9) |
| Total | 24 | 27 | 28 | 35 |
| In Table 10,<br>Value : Number of patients, and<br>( ) : %. | | | | |

(D) Improvement in algia at rest time

Changes in algia at rest time were shown in following Table 11 and there was found no significant difference between the groups, but improvements in L group were 17.2% (5/29) in the first week and 28.6% (8/28) in the second week, in the level of "middle" or more. While, in H group, those were 35.7% (10/28) in the first week and 44.1% (15/34) in the second week. When the judging standard was level downed to "light" or more, percentages increased to 41.4% (12/29) in the first week and 67.9% (19/28) in the second week for L group, and 60.7% (17/28) in the first week and 67.6% (23/34) in the second week for H group to find out that the kininogenase has an action for reducing the algia.

Table 11

| Item | After 1 week | | After 2 weeks | |
|---|---|---|---|---|
| | L group | H group | L group | H group |
| Remarkably improved | 1 ( 3.4) | 3 (10.7) | 5 (17.9) | 5 (14.7) |
| Improved in middle | 4 (13.8) | 7 (25.0) | 3 (10.7) | 10 (29.4) |
| Improved in light | 7 (24.1) | 7 (25.0) | 11 (39.3) | 8 (23.5) |
| Not changed | 17 (58.6) | 11 (39.3) | 9 (32.1) | 10 (29.4) |
| Aggravated | 0 ( 0) | 0 ( 0) | 0 ( 0) | 1 ( 2.9) |
| Total | 29 | 28 | 28 | 34 |
| In Table 11,<br>Value : Number of patients, and<br>( ) : %. | | | | |

(E) Improvement in shakes of limbs

Changes in shakes of limbs were shown in following Table 12 and there was found no significant difference between the groups, but improvements in L group were 16.7% (4/24) in the first week and 30.4% (7/23) in the second week, in the level of "middle" or more. While, in H group, those were 30.4% (7/23) in the first week and 31.0% (7/29) in the second week. When the judging standard was level downed to "light" or more, percentages increased to 41.7% (10/24) in the first week and 56.5% (13/23) in the second week for L group, and 47.8% (11/23) in the first week and 55.2% (16/29) in the second week for H group to find out that the kininogenase has an action for reducing the shakes of limbs.

Table 12

| Item | After 1 week | | After 2 weeks | |
|---|---|---|---|---|
| | L group | H group | L group | H group |
| Remarkably improved | 1 ( 4.2) | 0 ( 0) | 2 ( 8.7) | 1 ( 3.4) |
| Improved in middle | 3 (12.5) | 7 (30.4) | 5 (21.7) | 8 (27.6) |
| Improved in light | 6 (25.0) | 4 (17.4) | 6 (26.1) | 7 (24.1) |
| Not changed | 14 (56.0) | 12 (52.2) | 10 (43.5) | 13 (44.8) |
| Aggravated | 1 ( 4.2) | 0 ( 0) | 0 ( 0) | 0 ( 0) |
| Total | 24 | 23 | 23 | 29 |
| In Table 12, Value : Number of patients, and ( ) : %. | | | | |

(5) Improvement classified into items of background factors

Analysises by t-examination, U-examination and Fisher's direct probability calculation method on 66 cases recognized of usefulness showed that a significant difference or a tendency thereof ( $P < 0.05$ or less) between the groups L and H can be recognized in following parameters, and H group are excellent than L group excepting such a case that position of ulcer is leg (back of legs and heel).

Male in Sex distinction; Age (40 - 59); In-patients; middle in Symptom; lower limbs in Position of infarction; lower limbs (back of legs, foot and heel) in Position of ulcer; "yes" in Record of operations; "no" in Accompanying disease; "yes" in Drug used jointly; $\leq$ 10mm to < 20mm ($\sqrt{}$ major axis x $\sqrt{}$ minor axis) in Size of ulcer; somewhat malignancy in Condition of granulation; "yes" in Infection; and middle in Shakes of limbs.

(6) General examinations

Ankle pressure index, blood pressure and number of heart beats were measured to evaluate an influence of the drug thereto.

(A) Ankle pressure index

On L group, no significant difference was not recognized at the time after lapsed 1 week from the initiation of administration, but a significant increase from 0.53 ± 0.07 (mean ± SE) to 0.63 ± 0.07 was recognized at the time after lapsed 2 weeks (P < 0.05).

On H group, while, significant increases from 0.52 ± 0.05 (before the administration) to 0.53 ± 0.07 (after 1 week) and 0.58 ± 0.06 (after 2 weeks) were recognized (P < 0.05 and P < 0.05, respectively).

(B) Blood pressure

No significant change was recognized in both groups and both times after lapsed 1 and 2 weeks.

(C) Side effect

Followings are main side effects judged by doctors as "have a relation with the drug", "may have the relation" or "can not be said as having or not having the relation". Generation ratio of the side effects was 16.2% (6/37) in L group and 9.3% (4/43) in H group, and no significant difference was recognized in the groups. To 1 person in L group and 3 persons in H group, administration of drug was stopped due to their heavy side effect.

Increase in rumbling sound; oppression in chest; cephalagia • unsteady feeling; bleeding in nose; reduction of blood pressure; flushing in face; diarrhea • sudation; nausea • thermal feeling in whole body; and abdominal pain.

Similar judgement was given in certain items in crinical examinations, but t-examination thereon and having a correspondence between values in pre- and post-administration of the drug showed that the change is in minor and gives no influence.

**Claims**

1. A pharmaceutical preparation for improving cerebral functions, which comprises an effective amount of kininogenases derived from human urine, having a main band at a position in molecular weight of 41,000 and a sub-band in molecular weight of 31,000, when SDS-polyacryloamide gel electrophoresis is applied for, showing a molecular weight in the range of about 47,000 - about 55,000, when gel-filtration methods are applied for, as well as having isoelectric points of pI 3.92, 4.08 and 4.23 in its original solution, and a pharmaceutically acceptable carrier therefor.

2. A pharmaceutical preparation as claimed in Claim 1, further comprising a stabilizer selected from citric acid and pharmaceutically acceptable salts thereof.

3. A pharmaceutical preparation as claimed in Claim 2, wherein it comprises about 0.15 PNA units/ml of the kininogenase and 0.1 - 1.0% by weight of the stabilizer.

4. A pharmaceutical preparation for preventing or curing a skin disease, which comprises an effective amount of kininogenases derived from human urine, having a main band at a position in molecular weight of 41,000 and a sub-band in molecular weight of 31,000, when SDS-polyacryloamide gel electrophoresis is applied for, showing a molecular weight in the range of about 47,000 - about 55,000, when gel-filtration methods are applied for, as well as having isoelectric points of pI 3.92, 4.08 and 4.23 in its original solution, and a pharmaceutically acceptable carrier therefor.

5. A pharmaceutical preparation as claimed in Claim 4, further comprising a stabilizer selected from citric acid and pharmaceutically acceptable salts thereof.

6. A pharmaceutical preparation as claimed in Claim 2, wherein it comprises about 0.15 PNA units/ml of the kininogenase and 0.1 - 1.0% by weight of the stabilizer.